# EUROPÄISCHE PATENTANMELDUNG

(11) **EP 2 466 540 A1**
(43) Veröffentlichungstag der Anmeldung: **20.06.2012**
(21) Anmeldenummer: 10194883.4
(22) Anmeldetag: 14.12.2010
(51) Int. Cl.: G06Q 50/00, G06F 19/00

(54) **Verfahren zur Fernüberwachung und Fernwartung von medizinischen Analysatoren**

(71) Anmelder: Roche Diagnostics GmbH, 68305 Mannheim (DE); F.Hoffmann-La Roche AG, 4070 Basel (CH)
(72) Erfinder: Herzele, Arno, 2345 Brunn am Gebirge (AT)
(74) Vertreter: Margotti, Herwig Franz

(57) **Zusammenfassung**

Bei einem Verfahren (11) zur Fernüberwachung und Fernwartung von medizinischen Geräten (A) mittels eines Remote Control Systems (1) ist vorgesehen, dass in einem medizinischen Analysator (A) nach jeder durchgeführten Probenmessung oder Qualitätskontrollmessung ein Messprotokoll (MP) erstellt wird, das durch eine Zusammenstellung von Messergebnissen und/oder davon abgeleiteten Rechenwerten und/oder Eingabewerten der Probenmessung oder Qualitätskontrollmessung gebildet ist, wobei das Messprotokoll (MP) einer automatischen Validierung seines Inhalts gemäß einstellbarer Validierungsregeln (VR) unterzogen wird, und das Messprotokoll (MP), wenn es die in den Validierungsregeln (VR) festgelegten Bedingungen erfüllt, über eine Datenverbindung (19), insbesondere ein Computernetzwerk, an ein entferntes Informationssystem (20) übertragen wird, wogegen, wenn das Messprotokoll (MP) die in den Validierungsregeln (VR) festgelegten Bedingungen nicht erfüllt, es zurückbehalten wird, um von einem Operator bearbeitet zu werden, und nach erfolgter Bearbeitung erneut der automatischen Validierung unterzogen wird.

## Beschreibung

Die Erfindung betrifft ein Verfahren zur Fernüberwachung und Fernwartung von medizinischen Geräten mittels eines Remote Control Systems.

So ein Verfahren ist in dem Dokument DE 10 2006 036 832 A1 offenbart, bei dem ein oder mehrere Computertomographen von der Ferne aus gewartet werden. Jeder dieser Computertomographen weist mehrere Detektormodule auf, denen je eine Identifikationseinrichtung mit einer eindeutigen elektronischen Signatur zugeordnet ist. Von jedem Detektormodul werden die erfassten Messergebnisse samt der Signatur des jeweiligen Detektormoduls an eine Fehlerdiagnose-Instanz übermittelt. In der Fehlerdiagnose-Instanz werden die Messergebnisse analysiert, wobei festgestellt wird, ob die Messergebnisse den qualitativen Erwartungen entsprechen, oder ob gegebenenfalls eines der Detektormodule fehlerhaft ist. In diesem Fall kann der Austausch des fehlerhaften Detektormoduls veranlasst werden.

Bei dem bekannten Verfahren zur Fernüberwachung und Fernwartung von medizinischen Geräten hat sich als Nachteil erwiesen, dass dieses Verfahren nicht zur Verarbeitung und Weiterleitung von Messergebnissen medizinischer Analysatoren geeignet ist, die zur Messung von Proben von Patienten vorgesehen sind. Bei solchen medizinischen Analysatoren muss einerseits die Einsatzbereitschaft und Funktionsfähigkeit des medizinischen Analysators aus der Ferne überwacht werden und andererseits müssen auch die mit diesen Analysatoren gewonnenen Messergebnisse bezüglich möglicher Fehler geprüft werden, bevor sie an das die medizinische Analyse beauftragende Krankenhaus oder den Arzt zur weiteren Bearbeitung weitergeleitet werden können.

Der Erfindung liegt die Aufgabe zugrunde, ein Verfahren zur Fernüberwachung und Fernwartung von medizinischen Analysatoren mittels eines Remote Control Systems zu schaffen, bei dem die vorstehend angegebenen Nachteile vermieden sind.

Erfindungsgemäß wird diese Aufgabenstellung dadurch gelöst, dass in einem medizinischen Analysator nach jeder durchgeführten Probenmessung oder Qualitätskontrollmessung ein Messprotokoll erstellt wird, das durch eine Zusammenstellung von Messergebnissen und/oder davon abgeleiteten Rechenwerten und/oder Eingabewerten der Probenmessung oder Qualitätskontrollmessung gebildet ist, wobei das Messprotokoll einer automatischen Validierung seines Inhalts gemäß einstellbarer Validierungsregeln unterzogen wird, und das Messprotokoll, wenn es die in den Validierungsregeln festgelegten Bedingungen erfüllt, über eine Datenverbindung, insbesondere ein Computernetzwerk, an ein entferntes Informationssystem übertragen wird, wogegen, wenn das Messprotokoll die in den Validierungsregeln festgelegten Bedingungen nicht erfüllt, es zurückbehalten wird, um von einem Operator bearbeitet zu werden, und nach erfolgter Bearbeitung erneut der automatischen Validierung unterzogen wird.

Hierdurch ist der Vorteil erhalten, dass ein für medizinische Analysatoren maßgeschneidertes Verfahren erhalten ist, bei dem die Analysatoren sämtliche relevanten Informationen in Messprotokollen bereitstellen, die gemäß einstellbarer Validierungsregeln überprüft und nur dann weitergeleitet werden, wenn das jeweilige Messprotokoll den Validierungsregeln entspricht. Ein Messprotokoll kann hierbei sowohl Messergebnisse der jeweiligen Probenmessung oder Qualitätskontrollmessung, als auch von dem Analysator davon bereits abgeleitete Rechenwerte enthalten. Zusätzlich können von einem Operator des Analysators an dem Analysator eingegebene Eingabewerte der Probenmessung oder Qualitätskontrollmessung in dem Messprotokoll enthalten sein. Um die Qualität der Messungen mit dem Analysator auf dem geforderten hohen Level zu halten, werden in vorgegebenen Zeitabständen Qualitätskontrollmessungen mit bekannten Referenzproben zur Kontrolle der Qualität der Messergebnisse des Analysators durchgeführt. Die dabei ermittelten Messergebnisse der Qualitätskontrollmessungen werden (analog den Probenmessungen, welche mit Patientenproben durchgeführt werden) ebenfalls in einem Messprotokoll zusammengefasst und gemäß den Validierungsregeln überprüft. Diese können für Probenmessungen und Qualitätskontrollmessungen übereinstimmen, aber auch in bestimmten Anforderungen voneinander abweichen.

Wenn das von dem jeweiligen Analysator abgegebene Messprotokoll einer oder mehrerer der Validierungsregeln nicht entspricht, dann wird es für die manuelle Überprüfung durch einen Operator bereitgestellt und von diesem gegebenenfalls verändert, worauf neuerlich eine Validierung anhand der Validierungsregeln erfolgt. Vorteilhafterweise werden nur den Validierungsregeln entsprechende Messprotokolle über eine Datenverbindung an das entfernte Informationssystem des entsprechenden Krankenhauses oder Arztes übertragen. Hierdurch ist sichergestellt, dass nur gemäß den Validierungsregeln überprüfte Messergebnisse von Probenmessungen an das medizinische Personal gelangen. Ergibt die Validierung der Messergebnisse einer Qualtitätskontrollmessung eine Fehlfunktion des in dem Messprotokoll identifizierten Analysators, dann kann eine entsprechende Überprüfung oder Wartung des Analysators veranlasst werden.

Weiters hat es sich als vorteilhaft erwiesen, die Übertragung der Messprotokolle entsprechend einem der etablierten medizinischen Standardprotokolle durchzuführen, wobei insbesondere das ASTM oder HL7 Protokoll vorteilhaft ist. Hierdurch sind die für die Übertragung medizinischer Daten nötigen Sicherheitsvorkehrungen gegeben und das Verfahren kann einfach in bestehende medizinische Informationssysteme integriert werden.

Bei der Überprüfung anhand der Validierungsregeln können bereits Auswertungen von Datenfeldern bzw. Messergebnissen innerhalb der Datensätze des Messprotokolls durchgeführt werden. Von diesen Auswertungsergebnissen können vorteilhafterweise auch bereits vorgegebene Aktionen abgeleitet werden.

Diese und weitere vorteilhafte Ausgestaltungen des erfindungsgemäßen Verfahrens werden im Folgenden anhand der Figuren näher erläutert.
Figur 1 zeigt ein Remote Control System zur Fernüberwachung und Fernwartung von medizinischen Analysatoren.
Figur 2 zeigt eine erste Bildschirmansicht an einem Computer des Remote Control Systems gemäß Figur 1.
Figur 3 zeigt ein Flussdiagramm eines Verfahrens zur Fernüberwachung und Fernwartung von medizinischen Analysatoren, das mit dem Remote Control System gemäß Figur 1 abgearbeitet wird.
Figur 4 zeigt eine zweite Bildschirmansicht des Remote Control Systems gemäß Figur 1.
Figur 5 zeigt eine dritte Bildschirmansicht des Remote Control Systems gemäß Figur 1.
Figur 6 zeigt eine vierte Bildschirmansicht des Remote Control Systems gemäß Figur 1.

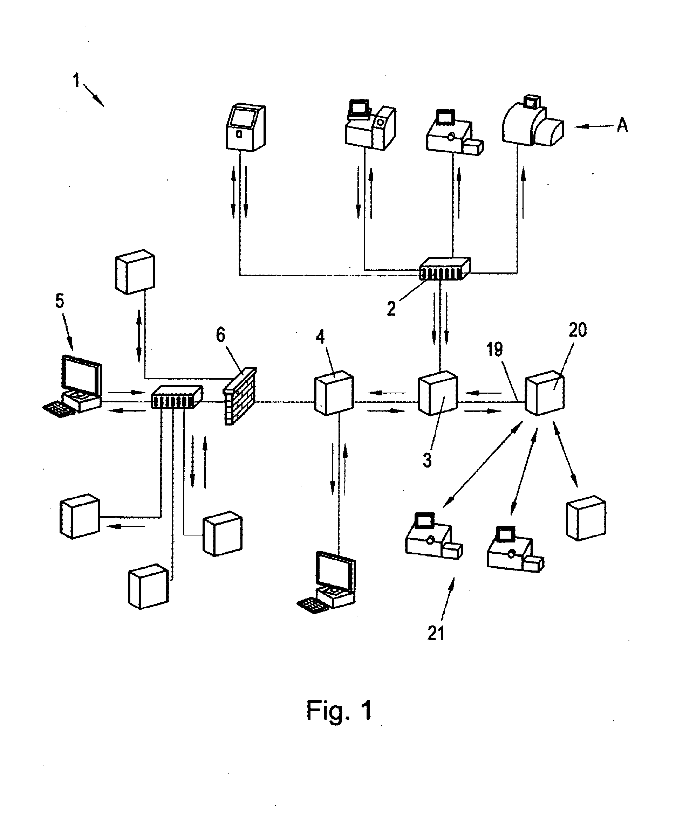

Figur 1 zeigt ein Remote Control System 1 zur Fernüberwachung und Fernwartung von Analysatoren A. Diese Analysatoren A sind in einem oder mehreren Krankenhäusern installiert und werden über das Remote Control System 1 in Echtzeit bezüglich Störungen und der Qualität und Zuverlässigkeit der Messergebnisse der Analysen überwacht. Die

Analysatoren A dienen zur dezentralen Probenmessung an Proben von Patienten der Krankenhäuser. Mit den Analysatoren A werden beispielsweise die Blutgaswerte (O₂, CO₂), der pH-Wert, bestimmte Elektrolytwerte (K⁺, Na⁺, Ca⁺⁺, Cl⁻) bestimmte Metabolitwerte (Glukose und Lactat), der Hämatokritwert, bestimmte Hämoglobinparameter (tHb, SO₂, etc.) oder der Bilirubinwert von Vollblutproben bestimmt. Dem Fachmann sind ein Vielzahl weiterer solcher Probenmessungen mit Analysatoren A bekannt, die ebenfalls in dem Remote Control System 1 durchgeführt werden könnten.

Die von dem jeweiligen Analysator A ermittelten Messergebnisse der Probenmessung oder Qualitätskontrollmessung werden von dem Analysator A in einem Messprotokoll festgehalten. Wenn ein Operator des Analysators A, der den Analysator A bei der Probenmessung bedient, Eingaben bezüglich der Patientendaten oder andere Eingaben zu der Probenmessung an dem Analysator A durchführt, dann werden diese Eingaben ebenfalls in dem Messprotokoll in den dafür vorgesehenen Datenfeldern festgehalten. Von dem Analysator A aus den Messergebnissen bereits abgeleitete Rechenwerte können ebenfalls in dem Messprotokoll enthalten sein. Um die Qualität der Probenmessungen mit dem Analysator auf dem gefordert hohen Level zu halten, werden in vorgegebenen Zeitabständen Qualitätskontrollmessungen mit bekannten Referenzproben zur Kontrolle der Qualität der Messergebnisse der Analysatoren A durchgeführt. Die dabei ermittelten Messergebnisse der Qualitätskontrollmessungen werden ebenfalls in dafür vorgesehene Datenfelder des Messprotokolls aufgenommen.

Das von dem jeweiligen Analysator A bei einer Probenmessungen oder bei einer Qualitätskontrollmessung erstellte Messprotokoll wird von dem Analysator A über einen Switch 2 und einen Verbindungscomputer 3 an einen Datenbankcomputer 4 übertragen und in diesem gespeichert. Von jedem Computer im Netzwerk, der über einen Internet Browser und entsprechende Zugangsberechtigungen verfügt, können die Messprotokolle aufgerufen und validiert werden. Diese Computer 5 können, wie in Figur 1 gezeigt, direkt mit dem Datenbankcomputer 4 verbunden sein, oder über ein Netzwerk, beispielsweise unter Berücksichtung einer Firewall 6.

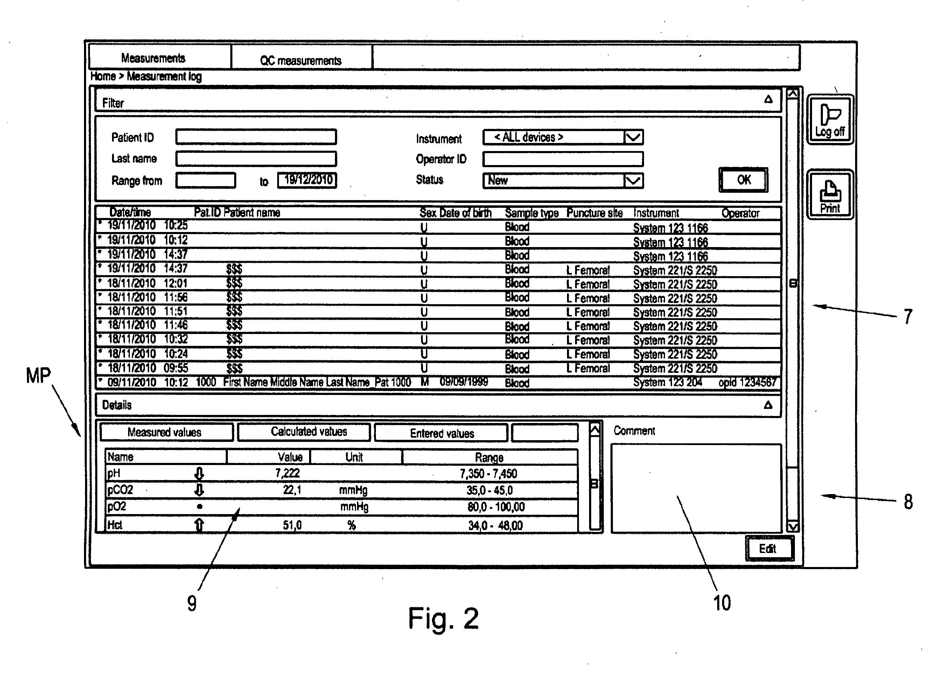

In Figur 2 ist eine erste exemplarische Bildschirmdarstellung zu sehen, die an dem Bildschirm des Computers 5 für einen Operator des Remote Control Systems 1 dargestellt wird. In einem oberen Bildschirmbereich 7 ist eine Liste mit Messprotokollen dargestellt und in einem unteren Bildschirmbereich 8 sind die Messergebnisse 9 und/oder Rechenwerte und/oder Eingabewerte sowie evtl. Anmerkungen in einem Anmerkungs-Datenfeld 10 des im Bildschirmbereich 7 selektierten Messprotokolls MP dargestellt. Zu jedem Messprotokoll MP sind in Figur 2 drei unterschiedliche Datenarten (Messergebnisse (Measured values), Rechenwerte (Calculated values), Eingegebene Werte (Entered values)) dargestellt. Der Operator erhält somit in Echtzeit einen Überblick über mit den Analysatoren A des Remote Control Systems 1 ermittelten Messprotokolle MP, die von ihm überprüft werden müssen.

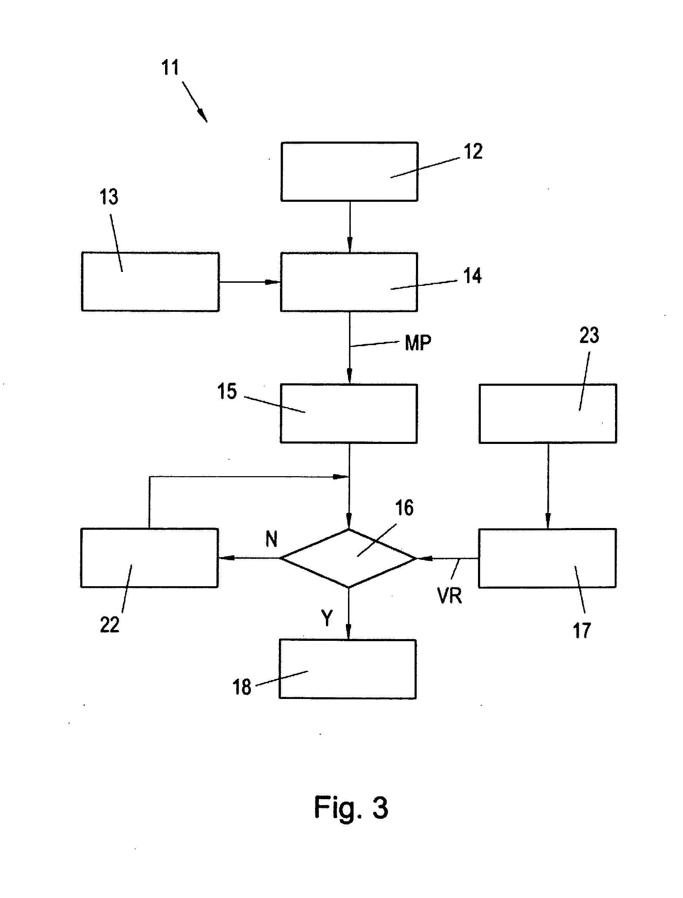

Figur 3 zeigt ein Flussdiagramm 11 eines Verfahrens zur Fernüberwachung und Fernwartung der medizinischen Analysatoren A des Remote Control Systems 1. Bei einem Block 12 des Flussdiagramms 11 wird mit einem der Analysatoren A eine Probenmessung oder Qualitätskontrollmessung durchgeführt und die gemessenen Messergebnisse und/oder davon abgeleiteten Rechenwerte werden von dem Analysator A in einem Messprotokoll MP gespeichert (Block 14). Bei einem Block 13 kann der Operator des Analysators A Eingaben an dem Analysator A durchführen, die dann bei einem Block 14 ebenfalls in dem Messprotokoll MP gespeichert werden.

Bei einem Block 15 wird das Messprotokoll MP von dem Analysator A über den Switch 2 und den Verbindungscomputer 3 an den Datenbankcomputer 4 übertragen und mit diesem gespeichert.

Für die Übertragung des Messprotokolls MP wird vorzugsweise ein medizinisches Standardprotokoll (beispielsweise ASTM-Protokoll) verwendet. Hierdurch sind die für die Übertragung medizinischer Daten in Messprotokollen MP nötigen Sicherheitsvorkehrungen gegeben und das Verfahren kann einfach in bestehende medizinische Informationssysteme integriert werden. Es hat sich auch als vorteilhaft erwiesen, mehrere solcher Standardprotokolle mit dem Remote Control System 1 zu unterstützen und je nach Gerätetyp des Analysators A eines dieser Protokolle an dem Verbindungscomputer 3 für die Kommunikation mit dem jeweiligen Analysator A auszuwählen.

Wenn der Operator des Validierungsmoduls an einem Computer 5 den entsprechenden Zugriff über einen Internet Browser öffnet und sich durch seinen Benutzer und Passwort identifiziert, dann erhält er die seiner Operator-ID zugeordneten Messprotokolle MP, beispielsweise entsprechend der ersten Bildschirmansicht in Figur 2, dargestellt. Hierbei kann beispielsweise festgelegt sein, dass jeder Operator eines Analysators A die Messprotokolle MP der von ihm durchgeführten Analysen als Operator des Validierungsmoduls angezeigt erhält. Ebenfalls könnte festgelegt sein, dass der Operator des Validierungsmoduls alle Messprotokolle MP sieht oder es könnte festgelegt sein, dass der Operator gerade jene Messprotokolle MP von Analysen sieht, die er nicht selber durchgeführt hat. Solche Einstellungen werden vorzugsweise von einem Administrator des Remote Control Systems 1 durchgeführt.

Bei dem Block 16 werden nunmehr die in der Liste enthaltenen Messprotokolle MP mit den gemäß Block 17 gespeicherten Validierungsregeln VR automatisch validiert. Zum Beginnen der Validierung kann es nötig sein, dass der Operator ein Schaltfeld in der Bildschirmanzeige aktiviert, oder die Validierung kann auch bereits völlig automatisch in Echtzeit unmittelbar nach dem Empfang des jeweiligen Messprotokolls MP von dem Verbindungscomputer 3 durchgeführt werden. Beim (automatischen) Validieren eines Messprotokolls MP wird von dem Verbindungscomputer 3 geprüft, ob die in den Datenfeldern des Messprotokolls gespeicherten Daten allen Validierungsregeln VR entsprechen. Wenn die Prüfung in Block 16 ergibt, dass alle Validierungsregeln VR erfüllt sind, dann wird in das Messprotokoll MP eingetragen, dass es mit den Validierungsregeln VR validiert wurde und diese somit erfüllt, worauf das validierte Messprotokoll MP bei einem Block 18 über eine Datenverbindung an einen entfernten Informationsserver übertragen wird. Bei diesem Informationsserver handelt es sich gemäß diesem Ausführungsbeispiel um einen Krankenhausserver 20, welcher über eine Datenverbindung 19 in dem das Messprotokoll MP gespeichert wird und von dem das Messprotokoll MP auf Endcomputer 21 von Ärzten und Schwestern des Krankenhauses herunter geladen werden kann.

Wenn hingegen die Prüfung bei Block 16 ergibt, dass zumindest eine der Validierungsregeln VR nicht erfüllt ist, dann wird dieses nicht validierte Messprotokoll MP zurückbehalten und nicht an den Informationsserver 20 übermittelt. Das Messprotokoll MP wird in diesem Fall in der Liste der ersten Bildschirmanzeige gemäß Figur 2 dargestellt, wohingegen validierte Messprotokolle MP in der Liste nicht mehr aufscheinen. Hierdurch ist der Vorteil erhalten, dass der Operator nicht alle von den Analysatoren A erstellten Messprotokolle MP durchsehen muss, sondern nur gezielt jene aufgelistet erhält, die problematisch sind, d.h. welche nicht alle vordefinierten Validierungsregeln erfüllen.

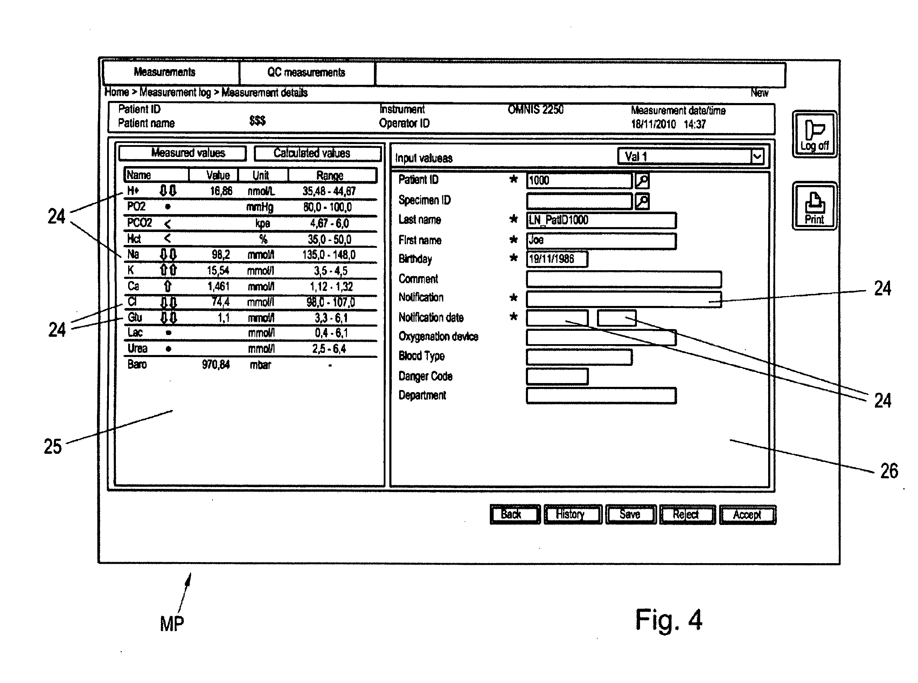

Der Operator kann nunmehr anhand einer zweiten Bildschirmansicht in Figur 4 die einzelnen Datenfelder des Messprotokolls MP überprüfen. Vorteilhafterweise werden jene Datenfelder 24 im Bildschirmbereich 25, die zumindest einer der Validierungsregeln VR nicht entsprochen haben, in der webbasierten Benutzeroberfläche farblich hervorgehoben dargestellt. Hierdurch kann der Operator sehr rasch erkennen, weshalb das Messprotokoll MP nicht automatisch validiert wurde, insbesondere welche Validierungsregeln nicht erfüllt wurden.

Manche der Datenfelder 24, nämlich insbesondere die Datenfelder 24 im Bildschirmbereich 25, des Messprotokolls MP kann der Operator nicht verändern, um vom System her sicherzustellen, dass Messergebnisse nicht einfach manuell überschrieben werden können. Andere Datenfelder 24, nämlich insbesondere die Datenfelder 24 im Bildschirmbereich 26, des Messprotokolls MP kann der Operator verändern, wenn ihm der Grund für den falschen Eintrag bekannt ist. Fehlende Datenfelder 24, wie beispielsweise Patientendaten im Bildschirmbereich 26, können von dem Operator ergänzt werden. Jede Veränderung des Messprotokolls MP durch den Operator wird gemeinsam mit der Operator-ID des Operators gespeichert, um Änderungen rückverfolgbar zu machen.

Wenn der Operator des Validierungsmoduls bei einem Block 22 das Messprotokoll MP durch entsprechende Eingaben verändert, dann wird dieses veränderte Messprotokoll MP neuerlich automatisch der Validierung im Block 16 zugeführt. Erfüllt das Messprotokoll MP nun alle Validierungsregeln VR, dann wird bei einem Block 18 des Verfahrens 11 das Messprotokoll MP über eine Datenverbindung an einen entfernten Informationsserver übertragen. Wenn das Messprotokoll MP andererseits wieder nicht alle Validierungsregeln VR erfüllt, dann bleibt das Messprotokoll MP in der Liste als "nicht validiert" gekennzeichnet erhalten. Diese nicht validierten Messprotokolle MP kann der Administrator überprüfen und festlegen, ob diese trotzdem mit entsprechenden Anmerkungen im Messprotokoll MP validiert werden sollen oder wie weiter vorzugehen ist. Hierdurch ist vorteilhafterweise sichergestellt, dass nur Messprotokolle MP, die allen Validierungsregeln VR entsprechenden, an den Informationsserver 20 übertragen werden, wodurch eine zuverlässige Datenqualität gewährleistet werden kann.

Bei einem Block 23 kann sich ein Supervisor über einen Computer des Remote Control Systems 1 mit seinem Benutzer und Kennwort identifizieren, worauf er, aufgrund seiner Zugriffsberechtigungen, dazu ermächtigt ist, Validierungsregeln VR neu aufzustellen oder bestehenden Validierungsregeln VR zu verändern.

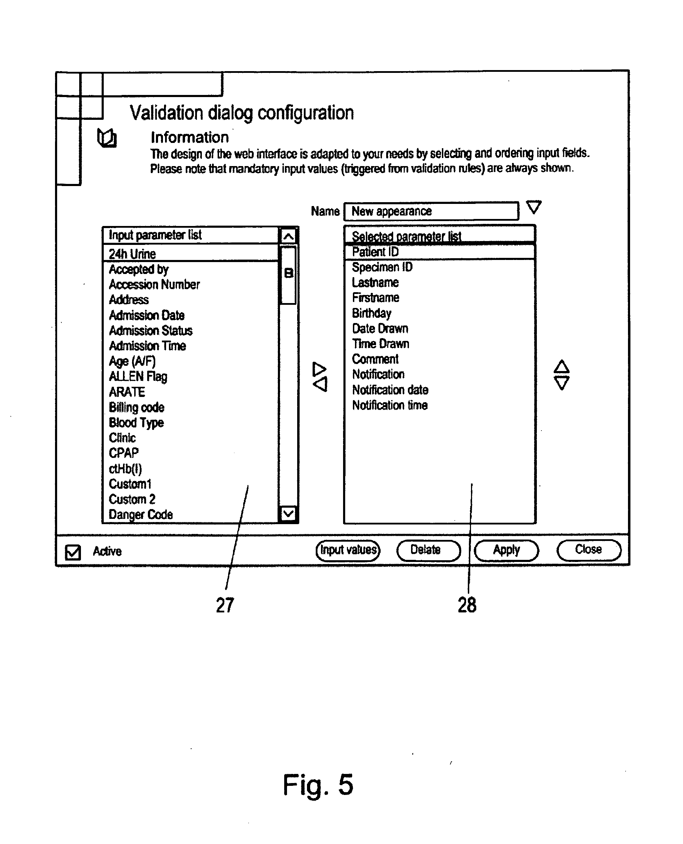

Nach dem Einloggen des Supervisors und der Auswahl eines entsprechenden Konfigurationsmoduls kann in einer dritten Bildschirmansicht gemäß Figur 5 die für das Validierungsmodul verwendete Anzeige des Validierungsdialogs konfiguriert werden. Hierfür können in einem Bildschirmbereich 27 jene Eingabefelder ausgewählt werden, die dann in dem Bildschirmbereich 26 der zweiten Bildschirmansicht gemäß Figur 4 angezeigt werden. In einem Bildschirmbereich 28 sind jene Eingabefelder dargestellt, die bei der Konfiguration in dem Bildschirmbereich 27 für die Anzeige ausgewählt wurden.

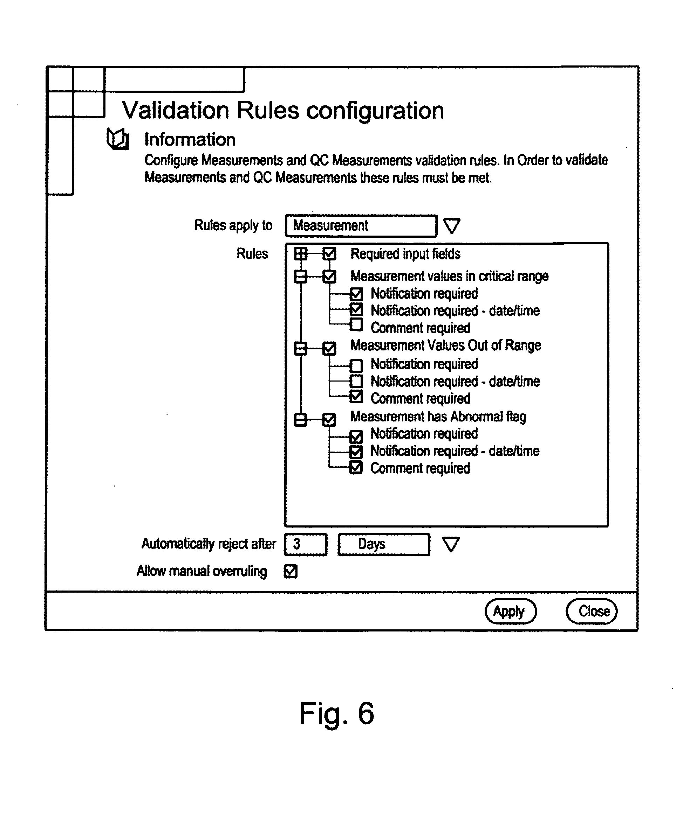

In einer vierten Bildschirmansicht gemäß Figur 6 werden die Validierungsregeln VR festgelegt. Hierbei können die gewünschten Regeln aus einer Liste durch Anhaken ausgewählt werden. Weiters kann festgelegt werden, dass eine Messung, die nicht innerhalb eines bestimmten Zeitraumes validiert worden ist, zurückgewiesen wird.

Bei der Eingabemaske gemäß Figur 6 können einzelne Validierungsregeln VR angehakt werden, um diese angehakten Validierungsregeln VR bei der Validierung der Messprotokolle MP zu prüfen. Im Folgenden werden beispielhaft die in Figur 6 angegebenen Validierungsregeln VR kurz erläutert:
Measurement Values in Critical Range: Ist der von dem Analysator gemessene Messwert innerhalb eines vorgegebenen zu erwartenden Messbereichs?
Notification required: Enthält das Messprotokoll eine Eingabe in dem Datenfeld der Warnmitteilungen, dass eine Warnmitteilung ausgesandt wurde, wenn der gemessene Messwert innerhalb eines vorgegebenen zu erwartenden Messbereichs ist?
Comment required: Enthält das Messprotokoll eine Eingabe in dem Datenfeld des Kommentars von einem z.B. verantwortlichen Arzt, wenn der gemessene Messwert innerhalb eines vorgegebenen zu erwartenden Messbereichs ist?
Measurement Values out of Range: Ist der von dem Analysator gemessene Messwert außerhalb eines vorgegebenen zu erwartenden Messbereichs?
Notification required: Enthält das Messprotokoll eine Eingabe in dem Datenfeld der Warnmitteilungen, dass eine Warnmitteilung ausgesandt wurde, wenn der gemessene Messwert außerhalb eines vorgegebenen zu erwartenden Messbereichs ist?
Comment required: Enthält das Messprotokoll eine Eingabe in dem Datenfeld des Kommentars von einem z.B. verantwortlichen Arzt, wenn der gemessene Messwert außerhalb eines vorgegebenen zu erwartenden Messbereichs ist?
Measurement has Abnormal Flag: Ist eine Probenmessung vom messenden Analysator mit der Kennzeichnung "Abnormale Messung" versehen?
Notification required: Enthält das Messprotokoll eine Eingabe in dem Datenfeld der Warnmitteilungen, dass eine Warnmitteilung ausgesandt wurde, wenn in dem Datenfeld für abnormale Probenmessungen oder Qualitätskontrollmessungen eine Information eingetragen ist?
Comment required: Enthält das Messprotokoll eine Eingabe in dem Datenfeld des Kommentars von einem z.B. Operator des Analysators, wenn in dem Datenfeld für abnormale Probenmessungen oder Qualitätskontrollmessungen eine Information eingetragen ist?

Anhand dieser Beispiele für Validierungsregeln VR ist dem Fachmann klar, dass je nach Typ des Analysators und zu erwartenden Messergebnissen weitere andere Validierungsregeln VR auswählbar oder vom Supervisor gegebenenfalls auch eingebbar sein können. Weiters kann in der Eingabemaske gemäß Figur 5 ausgewählt werden, ob diese Validierungsregeln VR auf Probenmessungen und/oder auf
Qualitätskontrollmessungen angewendet werden sollen.

Eine mögliche bevorzugte Validierungsregel VR ist die Überprüfung, ob vorbestimmte Aktionen durchgeführt wurden, wobei insbesondere überprüft wird, ob bei nicht vorhandenen Qualitätskontrollmesswerten oder bei Vorliegen vorbestimmter Abweichungen von Qualitätskontrollmesswerten oder bei Qualitätskontrollmesswerten, die in einem Bereich "Parameter lock" liegen, in einem bestimmten Datenfeld des Messprotokolls ein Kommentar eingetragen wurde. Als "Parameter lock" kann hierbei je nach Gerätetyp beispielsweise ein Bereich von größer oder gleich 2sd (statistische Standardabweichung) und kleiner 3sd oder aber auch größer oder gleich 3sd festgelegt sein. Durch das Validieren mit dieser Validierungsregel VR ist sichergestellt, dass, wenn die Messwerte einer Qualitätskontrollmessung auf ein Problem des Analysators A hinweisen, von dem Operator die geeigneten Schritte zur Lösung des Problems in die Wege geleitet werden. Beispielsweise könnte eine Wartung des Analysators A beauftragt werden oder aber auch der Analysator vorerst gesperrt werden, um das Risiko falscher Probenmessungen zu vermeiden.

Weiters kann in einer Validierungsregel VR bevorzugt festgelegt sein, dass bestimmte Aktionen, wie die Durchführung einer Wartung des Analysators A, innerhalb eines Zeitbereichs von beispielsweise drei Arbeitstagen durchgeführt werden müssen. Werden solche Aktionen innerhalb des vorgegebenen Zeitbereichs nicht durchgeführt und in dem System bestätigt, dann kann vorgesehen sein, dass Messergebnisse dieses Analysators A automatisch zurückgewiesen werden. Wenn also beispielsweise der bei einem Analysator A nötige Austausch eines Filters nicht innerhalb des vorgegebenen Zeitbereichs erfolgt und somit die Gefahr von verfälschten Messergebnissen gegeben ist, dann werden die Messprotokolle MP dieses Analysators A bei der Validierung gemäß dieser Validierungsregel VR nicht validiert

Weiters kann es vorteilhaft sein den Operator automatisch, also beispielsweise mittels E-Mail oder SMS, zu verständigen, wenn ein Messprotokoll MP nicht validiert wurde und von dem Operator geprüft werden muss. Hierdurch kann eine besonders rasche Bearbeitung von problematischen Messprotokollen MP sichergestellt werden.

## Patentansprüche

1. Verfahren (11) zur Fernüberwachung und Fernwartung von medizinischen Geräten (A) mittels eines Remote Control Systems (1), **dadurch gekennzeichnet, dass** in einem medizinischen Analysator (A) nach jeder durchgeführten Probenmessung oder Qualitätskontrollmessung ein Messprotokoll (MP) erstellt wird, das durch eine Zusammenstellung von Messergebnissen und/oder davon abgeleiteten Rechenwerten und/oder Eingabewerten der Probenmessung oder Qualitätskontrollmessung gebildet ist, wobei das Messprotokoll (MP) einer automatischen Validierung seines Inhalts gemäß einstellbarer Validierungsregeln (VR) unterzogen wird, und das Messprotokoll (MP), wenn es die in den Validierungsregeln (VR) festgelegten Bedingungen erfüllt, über eine Datenverbindung (19), insbesondere ein Computernetzwerk, an ein entferntes Informationssystem (20) übertragen wird, wogegen, wenn das Messprotokoll (MP) die in den Validierungsregeln (VR) festgelegten Bedingungen nicht erfüllt, es zurückbehalten wird, um von einem Operator bearbeitet zu werden, und nach erfolgter Bearbeitung erneut der automatischen Validierung unterzogen wird.

2. Verfahren (11) nach Anspruch 1, **dadurch gekennzeichnet, dass** die Übertragung des Messprotokolls (MP) an das Informationssystem (20) gemäß einem medizinischen Standardprotokoll, insbesondere gemäß einem ASTM oder HL7 Protokoll, erfolgt.

3. Verfahren (11) nach einem der vorhergehenden Ansprüche, **dadurch gekennzeichnet, dass** die Validierungsregeln (VR) Auswertungen innerhalb von Datensätzen der einzelnen Messprotokolle (MP) und Aktionen, die aus den Auswertungsergebnissen abgeleitet werden, umfassen.

4. Verfahren (11) nach Anspruch 3, **dadurch gekennzeichnet, dass** eine Validierungsregel (VR) festlegt, in welchen Datenfeldern der Messprotokolle (MP) von Probenmessungen und Qualitätskontrollmessungen Messergebnisse oder Rechenwerte oder Eingabewerte enthalten sein müssen.

5. Verfahren (11) nach Anspruch 3 oder 4, **dadurch gekennzeichnet, dass** auf die Messprotokolle (MP) von Probenmessungen anwendbare Validierungsregeln (VR) überprüfen, ob vorbestimmte Aktionen durchgeführt wurden, wobei insbesondere überprüft wird, ob bei nicht vorhandenen Messergebnissen oder Rechenwerten oder Eingabewerten oder bei Vorliegen vorbestimmter Abweichungen von Messergebnissen oder abgeleiteter Rechenwerte:
a) in einem bestimmten Datenfeld des Messprotokolls (MP) vermerkt wurde, dass ein Verantwortlicher vom Operator vom Messergebnis verständigt wurde, und/oder
b) in einem bestimmten Datenfeld des Messprotokolls (MP) ein Kommentar eingetragen wurde.

6. Verfahren (11) nach einem der Ansprüche 3 bis 5, **dadurch gekennzeichnet, dass** auf die Messprotokolle (MP) von Qualitätskontrollmessungen anwendbare Validierungsregeln (VR) überprüfen, ob vorbestimmte Aktionen durchgeführt wurden, wobei insbesondere überprüft wird, ob bei nicht vorhandenen Qualitätskontrollmesswerten oder bei Vorliegen vorbestimmter Abweichungen von Qualitätskontrollmesswerten oder bei Qualitätskontrollmesswerten, die in einem Bereich "Parameter lock" liegen, in einem bestimmten Datenfeld des Messprotokolls (MP) ein Kommentar eingetragen wurde.

7. Verfahren (11) nach Anspruch 3, **dadurch gekennzeichnet, dass** die durch die Validierungsregeln (VR) bedingten vorbestimmten Aktionen innerhalb eines bestimmten einstellbaren Zeitbereichs durchgeführt werden.

8. Verfahren (11) nach Anspruch 3, **dadurch gekennzeichnet, dass** die von den Validierungsregeln (VR) bedingten, nicht innerhalb des bestimmten einstellbaren Zeitbereichs durchgeführten Aktionen ein automatisches Zurückweisen der Messergebnisse bewirken.

9. Verfahren (11) nach Anspruch 8, **dadurch gekennzeichnet, dass** zurückgewiesene Aktionen von einem Administrator nachträglich validiert werden können.

10. Verfahren (11) nach einem der vorhergehenden Ansprüche, **dadurch gekennzeichnet, dass** Validierungsregeln (VR) für Messergebnisse und/oder Rechenwerte und/oder Eingabewerte von einem Supervisor festgelegt werden, wobei insbesondere vorgesehen ist, dass der Supervisor sich mit seinem Namen und/oder einem Kennwort für die Einstellung von individuellen Validierungsregeln (VR) identifizieren muss und nur nach erfolgter Identifizierung die Einstellung vornehmen kann, wenn er über eine ausreichende Berechtigung dafür verfügt, wobei insbesondere vorgesehen ist, dass die Einstellung von Validierungsregeln (VR) für Probenmessungen und Qualitätskontrollmessungen getrennt vorzunehmen sind.

11. Verfahren (11) nach einem der vorhergehenden Ansprüche, **dadurch gekennzeichnet, dass** dem Operator eine, insbesondere webbasierte Benutzeroberfläche für die Bearbeitung von Messprotokollen (MP), die in den Validierungsregeln (VR) festgelegte Bedingungen nicht erfüllt haben, bereitgestellt wird, wobei insbesondere vorgesehen ist, dass Messergebnisse, welche die Validierungsregeln (VR) nicht erfüllen, in der Benutzeroberfläche insbesondere farblich hervorgehoben werden.

12. Verfahren (11) nach Anspruch 10 oder 11, **dadurch gekennzeichnet, dass** einem Operator nur jene Messprotokolle (MP) von Messungen zur Bearbeitung zur Verfügung gestellt werden, die er selbst durchgeführt hat.

13. Verfahren (11) nach einem der vorhergehenden Ansprüche, **dadurch gekennzeichnet, dass** ein Operator automatisch, insbesondere mittels E-Mail verständigt wird, wenn ein Messprotokoll (MP) die in den Validierungsregeln (VR) festgelegten Bedingungen nicht erfüllt.

14. Verfahren (11) nach einem der vorhergehenden Ansprüche, **dadurch gekennzeichnet, dass** das Informationssystem ein Labor- oder Krankenhausinformationssystem ist.
